# EUROPEAN PATENT APPLICATION

(11) **EP 4 190 236 A1**
(43) Date of publication of application: **07.06.2023**
(21) Application number: 21211962.2
(22) Date of filing: 02.12.2021
(51) Int. Cl.: A61B 5/11, A61B 5/00

(54) **METHOD AND APPARATUS FOR DETERMINING A SPATIAL CONFIGURATION OF A WIRELESS INDUCTIVE NETWORK AND FOR POSE DETECTION**

(71) Applicant: ETH Zürich, 8092 Zürich (CH)
(72) Inventor: WITTNEBEN, Armin, 8044 Zürich (CH); SCHULTEN, Henry, 8050 Zürich (CH)
(74) Representative: Frei Patent Attorneys

(57) **Abstract**

The method for determining a spatial configuration of a wireless inductive network (N) comprising at least one active node (Al) and a first passive node (PI) and a second passive node (P2) comprises
- generating a probe signal in the active node producing a probe electromagnetic field;
- inductive interaction in response to the probe electromagnetic field contributing to a back-coupled electromagnetic field, the inductive interaction involving the active node, the first passive node and the second passive node;
- the back-coupled electromagnetic field superposing with the probe electromagnetic field and inducing in the active node a back-coupled signal;
- the back-coupled signal superposing in the active node with the probe signal to result in a measurement signal;
- measuring one or more properties of the measurement signal to determine a measurement value for each of the properties;
- deriving measurement data from the so-determined one or more measurement values;
- determining the spatial configuration in dependence of the measurement data.

Each of the nodes comprises an oscillating circuit. The resonance curves of the first and second passive nodes (PI, P2) can overlap. Cost-efficient and fast pose detection can be realized.

## Description

The invention relates to the field of sensing, more particularly of position and/or orientation sensing, more particularly of inductively sensing relative positions and/or orientations. It relates to methods and apparatuses according to the opening clauses of the claims. The invention can find application, e.g., in pose sensing, such as where relative positions and/or orientations of different parts of a person's or an animal's body are to be determined, or in robotics, such as to determine relative movements of different parts of a robot.

From WO2007/009088, a method and an apparatus for detecting object orientation and position is known. The apparatus includes a plurality of relatively complex sensors.

In US2020/0397345, a method and an apparatus for human activity recognition is known in which a receiver coil and a plurality of transmitter coils are mounted at different locations on a subject. Each transmitter coil is inductively coupled to the receiver coil, and from time-dependent signals, different motions can be inferred.

One object of the invention is to create a way of determining positions and/or orientations which can be implemented in a relatively simple way. In particular, a corresponding method and a corresponding combination for determining a spatial configuration of a wireless inductive network shall be provided as well as a corresponding method for determining a relative spatial configuration of a first item and a second item. These can be used for determining object orientation, such as for detection of a pose (bodily attitude) of a person or of an animal.

Another object of the invention is to provide a way of determining positions and/or orientations which can be implemented in a relatively cost-effective way.

Another object of the invention is to provide a way of determining positions and/or orientations which produces results relatively rapidly.

Another object of the invention is to provide a create a way of determining positions and/or orientations which produces results already after a very small number of measurements.

Another object of the invention is to provide a way of determining positions and/or orientations which can be flexibly implemented, in particular wherein components of the system can be particularly small.

Another object of the invention is to provide a way of determining positions and/or orientations which is rather insensitive to presence of various materials, such as of biological materials.

Further objects and various advantages emerge from the description and embodiments below.

At least one of these objects can be achieved in some implementations of apparatuses and/or methods described in this disclosure.

Inductive interactions in a wireless inductive network involving two nodes can be complicated, but can be understood and handled quite well. For example, providing a transmitter coil as active node of the network and another coil as a passive node of the network, it is possible to infer information about the relative position and/or orientation of these nodes from their inductive interaction taking place when a probe signal, such as a sine voltage, is a applied to the active node, and the resulting current in the active node is measured. The probe signal produces a probe electromagnetic field which inductively couples to the passive node, thus inducing a voltage and a current in the passive node. The inductive interaction leads to a back-coupled electromagnetic field which superposes with the probe electromagnetic field and induces in the active node a back-coupled signal. The back-coupled signal depends on the relative position and/or orientation of the nodes, such that said resulting current can also depend on the relative position and/or orientation of the nodes.

The system remains relatively simple even in case of more than one active nodes and more than one passive nodes, if the inductive interactions are limited to involving two nodes at a time (an active one and an inactive one). This can be accomplised, e.g., by selecting a frequency of the probe signal (excitation frequency) at which only a single passive node reacts, such as by selecting an excitation frequency close to the resonance frequency of said single passive node, while resonance frequencies of all other passive nodes are far distant from the excitation frequency and have sharp (low-bandwidth) resonance peaks. Simultaneous measurements are possible in this case, namely, e.g., by operating two active nodes at two sufficiently different excitation frequencies, the one interacting only with said single passive node, the other interacting with another single passive node.

Inductive interactions in a wireless inductive network involving three or more nodes can be and often are very complex. It is believed that such interactions are avoided in prior art because the system in this case can be difficult to understand and measurement data can be difficult to interpret or evaluate.

The inventors, however, realized that such complex inductive interactions can be very helpful in determining positions and/or orientations. Even if the evaluation of measurement data can be complex, the additional information gained from the additional passive node(s) is valuable. From a single measurement (at a single excitation frequency, at a single time), information from two or more passive nodes interacting with the active node and possibly also interacting among each other can be derived, thus making possible to better distinguish different spatial configurations of the network, i.e. different relative positions and/or orientations of the nodes.

In particular, the inventors contemplated the following method for determining a spatial configuration of a wireless inductive network, wherein the network comprises at least three nodes; the nodes comprising an active node and at least two passive nodes comprising a first passive node and a second passive node. The method comprises
- generating a probe signal in the active node producing a probe electromagnetic field;
- inductive interaction in response to the probe electromagnetic field contributing to a back-coupled electromagnetic field, wherein the inductive interaction involves the active node, the first passive node and the second passive node;
- the back-coupled electromagnetic field superposing with the probe electromagnetic field and inducing in the active node a back-coupled signal;
- the back-coupled signal superposing in the active node with the probe signal to result in a measurement signal;
- measuring one or more properties of the measurement signal to determine a measurement value for each of the properties;
- deriving measurement data from the so-determined one or more measurement values;
- determining the spatial configuration in dependence of the measurement data.

Passive nodes can be very simple, small and cost-effective. Furthermore, already a single measurement can produce a useful result. And a single measurement can be accomplished within a very short time duration.

The term network as herein used does not refer to merely an abstract topology, but to items (nodes), i.e. to the collection of interacting items (nodes).

Being an inductive network, the nodes are inductively coupled, i.e. they can interact by induction, more precisely by electromagnetic induction.

Being an wireless network, the nodes of the network are purely (merely) wirelessly coupled. With the nodes merely wirelessly coupled, the nodes of the network are, more specifically, galvanically isolated from one another.

Being an wireless network, each of the nodes of the network can be embodied as a circuit which is separate from the circuit by which any other one of the nodes is embodied.

Each of the nodes of the network can comprise and in particular be embodied as an electronic circuit, more particularly an oscillating circuit.

The oscillating circuit can in particular be an LC resonator, i.e. it can comprise an inductance, such as a coil, and a capacitance. In practice, there will also be an ohmic resistance, however in instances, the ohmic resistance will merely be the unavoidable ohmic resistance of the circuit, e.g., comprising the ohmic resistance of the coil. The capacitance comprises the unavoidable capacitance of the circuit, e.g., comprising the unavoidable capacitance of the coil, however, the oscillating circuit can in addition comprise a capacitor, such as for tuning the oscillating circuit to a suitable frequency (resonance frequency) - as is well known in electronics.

In case the inductance of a node is embodied to comprise a coil, that coil can be implemented, e.g., in form of a wire, but can also be implemented in a microstructured way, such as in form of a MEMS (microelectromechanical system). The coil can have less than one turn, but typically has at least one turn. For increased inductive interaction, it can be advantageous to provide that the coil has more than one turn, in particular in case of passive nodes; e.g., at least two turns.

In a particularly simple and cost-effective embodiment, at least one of the passive nodes, and in particular each of the passive nodes, is embodied as a circuit comprising merely a capacitor and a coil, e.g., a wound piece of closed-loop wire.

The spatial configuration of the network can more specifically be understood as a relative spatial configuration of the nodes of the network. The (relative) spatial configuration can be described, e.g., by the relative positions and orientations in space of the nodes, such as by the 6D pose data of all the nodes, in particular with respect to one and the same coordinate system. Remark: "Pose data" refers to position an orientation data; it does not refer to a "pose" as used herein, in the sense of a bodily attitude, even though pose data can be used to describe a pose of a body, such as by specifying position and orientation of several parts of the body.

E.g., the spatial configuration of a network with merely three nodes can be specified by pose data, the pose data comprising 18 numbers (scalars): three cartesian coordinates and three angles of rotation for each of the three nodes.

However, the (relative) spatial configuration can also be described differently, e.g., less comprehensively, such as merely by positions, such as specified by 3D coordinates; or merely by orientations. Or it can be described still more simply, e.g., in a summarizing fashion, such as by assigning an identifier to certain (pre-determined) spatial configurations (e.g., calibration spatial configurations, cf. below) or to a group of similar spatial configurations. In the latter way (using such an identifier), the high.dimensional description of positions and/or orientations of all nodes need not be known or determined and can be replaced by an identifier which can be low-dimensional, e.g., can be a scalar.

If the active node comprises a coil, that coil can be considered a transmitter coil, as the probe electromagnetic field is emitted by the coil.

The active node can be connected to the measuring unit, in particular, it can comprise a connector for that purpose.

In some embodiments, the active node comprises a connector for application of the probe signal.

The passive nodes, however, usually can be devoid a connector.

In some embodiments, the first and the second passive nodes are devoid a connector for application of a probe signal.

In some embodiments, the first and the second passive nodes are devoid a connector for a galvanic connection to a power supply.

The passive nodes can in particular be devoid of active electronic components; or, at least, they can be devoid active electronic components which would be active during the method or more particularly during generating the probe signal in the active node.

The probe signal can be a periodic signal, such as a sine signal. It can have a constant amplitude.

The probe signal can more specifically be a signal comprising an excitation frequency component, i.e. a component at an excitation frequency, in particular wherein the excitation frequency component can constitute a main frequency component of the probe signal, i.e. the frequency component having the largest amplitude of all frequency components of the probe signal. For example, the probe signal can be a sine signal at the excitation frequency.

A time duration of the probe signal can be, e.g., below 10 s, more particularly below 1 s; and, for example, at least 0.1 ms or at least 0.5 ms.

The excitation frequency can be, e.g., between 10 kHz and 20 MHz, more specifically between 50 kHz and 2 MHz, still more specifically between 100 kHz and 1 MHz. For excitation frequencies below approximately 1.5 MHz or 1 MHz, external effects, such as from biological tissues and living bodies, are small or even negligible. For frequencies below 10 kHz or 50 kHz, magnetic flux changes produced by the probe electromagnetic field tend to be so small that the back-coupled signal is very small and that the measurement signals are difficult or even impossible to analyze. Higher flux changes achievable at high excitation frequencies tend to result in larger back-coupled signals, but electronics for signal analysis needs to be more elaborate and more expensive, such that excitation frequencies below approximately 2 MHz or 1 MHz can be preferred.

The inductive interaction contributing to the back-coupled electromagnetic field and which, at least in absence of further interacting nodes in addition to the described three nodes, produces the back-coupled electromagnetic field, involves said three nodes (the active node and the two passive nodes). Thus, the active node, the first passive node and the second passive node participate in said inductive interaction.

In other words, the active node, the first passive node and the second passive node react to the probe electromagnetic field due to inductive interaction.

This is in contrast to setups in which only a single passive node inductively interacts with an active node (at a time, in reaction to a single probe signal).

Both, the first passive node and the second passive node, more specifically, the active node and the first passive node and the second passive node, can contribute to the back-coupled signal. By "contributing", we more particularly mean that the contribution is measurable (or rather measured), in particular such that the measurement data depend on (are influenced by) the presence of the respective nodes. All this more specifically, in reaction to a single probe signal.

Typically, the spatial configuration remains essentially constant during a measurement, such as during the duration of a single probe signal; strong changes of the spatial configuration might distort the measurement results. In this regard, the measurement can be referred to as static measurements, such as measurements of static poses. Yet, the spatial configuration can change during a measurement; in this case, e.g., a time-averaged spatial configuration, averaged over the measurement time, can be determined.

The interactions between the nodes are usually very complex, as they comprise many interactions. E.g., the probe electromagnetic field induces a current in the first node producing a voltage in the first node which produces a secondary electromagnetic field which inductively influences the active node (and possibly also the second passive node...), and the corresponding changes in the active node again change how the active node influences the first passive node, and so on - and the effects of the second passive node still add up. However, all this happens at the speed of light and thus practically instantaneously. E.g., provided the probe signal has a constant (static) frequency and amplitude, and the spatial configuration is static, also the back-coupled electromagnetic field, the back-coupled signal, and the measurement signal can all be expected to have a constant (static) frequency and amplitude each - during the duration of the probe signal.

Typically, the measurement signal is bound to depend on, i.e. is bound to be influenced by, all the three nodes, in particular by the first passive node and by the second passive node.

Accordingly, the measurement signal can depend on a presence of both, the first and the second passive node. More particularly, the measurement data can depend on a presence of both, the first passive node and the second passive node. In other words, an absence of any one of the first and second passive nodes would lead to a different measurement signal and more particularly to different measurement data. In particular, an absence of any one of the first and second passive nodes would lead to a measurable changes of the measurement signal.

Based on an analysis of the measurement signal, information can be gained regarding the spatial configuration.

For analyzing the measurement signal, said one or more properties of the measurement signal are measured.

In some embodiments, one the one or more properties comprise at least one of: a phase, an amplitude; in particular both.

In some embodiments, one of the properties is an amplitude of the measurement signal. Said amplitude can be, e.g., a voltage amplitude or a current amplitude. For example, in case the probe signal has a constant current amplitude, one of the properties can be a voltage amplitude, the measurement value corresponding to a figure of volts; or in case the probe signal has a constant voltage amplitude, one of the properties can be a current amplitude, the measurement value corresponding to a figure of amperes.

In some embodiments, one of the properties is a phase of the measurement signal, e.g., a phase difference (phase shift) between a current signal of the measurement signal and a voltage signal of the measurement signal, the measurement value corresponding to a figure of degrees.

In some embodiments, one of the properties is an impedance (effective impedance) of the network. This can be measured using various methods, e.g., well known Direct I-V (Direct current-voltage) measurements, ABB (Auto-balanced bridge) measurements, RF-IV (Radio frequency current-voltage) measurements.

Deriving the measurement data from the one or more measurement values can be trivial: the measurement data can be identical to a set composed of the one or more measurement values. Or the deriving can, e.g., include some calculations or the like, e.g., deriving an absolute value of an impedance from current and voltage values, or deriving a complex impedance from current and voltage values and a phase difference value.

With the aid of the measurement data, the spatial configuration can be determined. This does not mean that a single measurement is necessarily suffient to sufficiently precisely determine the spatial configuration. However, for example, it can be possible that with the aid of the measurement data, a number of possible spatial configurations can be reduced. Carrying out further measurements, e.g., at different excitation frequencies and/or including further passive nodes, and possibly also including one or more further active nodes, can refine the evaluation and lead to more precise results.

In particular, determining the spatial configuration can be based on classification, as will be described below in more detail.

In some embodiments,
- inductive interaction between the active node and the first passive node in response to the probe electromagnetic field; and
- inductive interaction between the active node and the second passive node in response to the probe electromagnetic field;
contribute to the back-coupled electromagnetic field superposing with the probe electromagnetic field and inducing in the active node the back-coupled signal.

In particular, both,
- the contributing of the inductive interaction between the active node and the first passive node to the back-coupled electromagnetic field; and
- the contributing of the inductive interaction between the active node and the second passive node to the back-coupled electromagnetic field;
contribute to the measurement data. With reference to explanations above referring to the term "contributing", they more specifically contribute to the measurement data to an extent which is measurable, i.e., more particularly, the measurement data depend on these contributions.

As will be readily understood, this way, the measurement data can contain more information than if only a single passive node would contribute.

Even more complex is the case that inductive interaction between the first passive node and the second passive node contributes, to the back-coupled electromagnetic field and more particularly to the measurement data:
In some embodiments, inductive interaction between the first passive node and the second passive node in response to the probe electromagnetic field contributes to the back-coupled electromagnetic field; and more particularly contributes to the measurement data.

As will be readily understood, this way, the measurement data can contain more information than if only a single passive node would contribute.

This can make possible to access passive nodes which are otherwise not accesible, e.g., in case the probe electromagnetic field would not interact with the second passive node because it is located too far away from the active node to contribute to the back-coupled electromagnetic field, or is oriented in a way that induction in response to the probe electromagnetic field is not sufficiently large to result to a contribution to the back-coupled electromagnetic field.

In the former case (location of second passive node too far away), a measurement distance range of the method can be effectively extendable by positioning the first passive node between the active node and the second passive node, similar to a waveguiding effect.

In the latter case (orientation of the second passive node) a suitably oriented and positioned first passive node can make possible that the second passive nodes influences the measurement via inductive interaction of the first passive node with both, the active node and the second passive node.

In some embodiments,
- the first passive node comprises a first oscillating circuit exhibiting a resonance behavior characterized by a first resonance curve comprising a first resonance peak at a first resonance frequency; and
- the second passive node comprises a second oscillating circuit exhibiting a resonance behavior characterized by a second resonance curve comprising a second resonance peak at a second resonance frequency.

In many instances, an overlap of the resonance curves of the passive nodes can be useful to make sure that the inductive interaction involves the active node, the first passive node and the second passive node. However, considering, e.g., effects of mutual induction, it is possible that the inductive interaction involves the active node, the first passive node and the second passive node even in cases where the resonance curves of the passive nodes do not overlap.

However, in some embodiments, the first and second resonance peaks overlap one another.

In particular, the resonance curve can describe, as well known in the art, the current-frequency characteristic measured at a constant voltage.

In case the overlapping of resonance curves needs to be defined more specifically, it can be defined using well-known dB values:
In some embodiments, twice a difference between the first resonance frequency and the second resonance frequency is identical to or smaller than a sum of a 40 dB width of the first resonance peak and a 40 dB width of the second resonance peak, more particularly smaller than a sum of a 30 dB width of the first resonance peak and a 30 dB width of the second resonance peak, still more particularly smaller than a sum of a 20 dB width of the first resonance peak and a 20 dB width of the second resonance peak.

The term "difference" when herein referring to a difference between two (resonance) frequencies more specifically refers to the absolute value of the difference between the (resonance) frequencies.

For example, in case of the 30 dB case above, for each of the two resonance peaks the width of the peak is taken where the current amplitude is 1/31.62 of the current amplitude at the resonance frequency, i.e. at the maximum of the resonance peak.

Based on a preferred alternative for more specifically defining the overlapping of resonance curves, which better takes into account peculiarities of asymmetric resonance curves, the following can be the case:

In some embodiments, the first resonance frequency is lower than the second resonance frequency, and a difference between the first resonance frequency and the second resonance frequency is identical to or smaller than a sum of an upper half-width of the first resonance peak and a lower half-width of the second resonance peak,
- the upper half-width of the first resonance peak being a difference between the first resonance frequency and a frequency larger than the first resonance frequency at which the first resonance curve assumes a value of 1 % of a value of the first resonance curve at the first resonance frequency;
- the lower half-width of the second resonance peak being a difference between the second resonance frequency and a frequency smaller than the second resonance frequency at which the second resonance curve assumes a value of 1 % of a value of the second resonance curve at the second resonance frequency.

This roughly corresponds to the 40 dB case above.

More particularly,
- the upper half-width of the first resonance peak being a difference between the first resonance frequency and a frequency larger than the first resonance frequency at which the first resonance curve assumes a value of 10 % of a value of the first resonance curve at the first resonance frequency;
- the lower half-width of the second resonance peak being a difference between the second resonance frequency and a frequency smaller than the second resonance frequency at which the second resonance curve assumes a value of 10 % of a value of the second resonance curve at the second resonance frequency.

This roughly corresponds to the 20 dB case above.

Similarly, an overlap at 3.162 % can be defined, roughly corresponding to the 30 dB case above.

As has been indicated above, the probe signal can comprise an excitation frequency component (at the excitation frequency).

In many instances, it can be useful if the excitation frequency lies within at least one of the resonance peaks of the first and second passive nodes, respectively. This can, in instances, increase the likelihood that the inductive interaction involves the active node, the first passive node and the second passive node. However, considering the effects of mutual induction, it is possible that the inductive interaction involves the active node, the first passive node and the second passive node even in cases where the excitation frequency does not lie within one of the resonance curves.

However, in some embodiments, the probe signal comprises an excitation frequency component, the excitation frequency component having an excitation frequency lying within one or both of: the first resonance peak and the second resonance peak. In particular, it can lie within both said resonance peaks.

In case the lying of the excitation frequency within a resonance peak needs to be defined more specifically, it can be defined as follows:

The respective resonance curve assumes at the excitation frequency a value of at least 1 %, more particularly of at least 3.162 %, still more particularly of at least 10 %, of a value of the resonance curve at the respective resonance frequency.

In view of the complexity of the inductive interaction, in particular if still further passive nodes are involved therein (in addition to the active node and the first passive node and the second passive node), a suitable way of determining the sought spatial configuration can be based on classification. In particular:
In some embodiments, the method comprises
- providing calibration data comprising, for each of a plurality of calibration spatial configurations of the network, calibration measurement data associated with a respective calibration spatial configuration; and
determining the spatial configuration in dependence of the measurement data comprises
- carrying out a classification step in which the spatial configuration is classified with the aid of the calibration data and in dependence of the measurement data.

The calibration data can be derived in various ways, e.g., based on measuments, such as bringing the network into a calibration spatial configuration and deriving the associated calibration measurement data, in particular including generating the probe signal, determining resulting measurement value(s) from the associated measurement signal; and doing so for all the calibration spatial configuration of the calibration data or for a portion of the calibration spatial configurations. It is also possible to derive the calibration data from simulations. Furthermore, it is also possible to derive a portion of the calibration data by interpolation between calibration spatial configurations and associated calibration measurement data, which were derived, e.g., from measurements or from simulations.

Determining the spatial configuration in dependence of the measurement data can comprise, e.g., comparing the measurement data to the calibration measurement data.

In some embodiments, the classification step comprises
- selecting, in dependence of the measurement data, one of the calibration measurement data;
- classifying the spatial configuration in dependence of a calibration spatial configuration which is associated with the selected calibration measurement data.

For example, those calibration measurement data can be selected which are most similar to the measurement data, such as those calibration measurement which differ least from the measurement data. The selecting can also be carried out based on machine learning.

Furthermore, in particular, the classifying the spatial configuration in dependence of a calibration spatial configuration which is associated with the selected calibration measurement data can comprise: classifying the spatial configuration to be the calibration spatial configuration which is associated with the selected calibration measurement data. The classifying can also be carried out based on machine learning.

In some embodiments, the classification step is carried out based on machine learning.

The described method can, of course, be applied for various purposes, such as for determining a spatial configuration of items or for determining a pose of an item (pose of a body). Nodes of the network can be positioned relative to the item(s) for this.

The spatial configuration of items can be defined analogously to the spatial configuration of a network (cf. above). For example, a spatial configuration of items can more specifically be understood as a relative spatial configuration of the items. The (relative) spatial configuration can be described, e.g., by the relative positions and orientations in space of the items, such as by the 6D pose data of all the items, in particular with respect to one and the same coordinate system. And it can be described, e.g., in a summarizing fashion, such as by assigning an identifier to certain (pre-determined) spatial configurations (e.g., calibration spatial configurations).

Generally, the method can be applied for determining a relative spatial configuration of a first item and a second item. The items, e.g., may be separate items or may be different parts of a single entity.

More particularly, we describe a method for determining a relative spatial configuration of a first item and a second item, wherein the method comprises
- providing a network which is a wireless inductive network comprising at least three nodes, the nodes comprising an active node and at least two passive nodes comprising a first passive node and a second passive node;
- positioning at least one of the nodes relative to the first item;
- positioning at least another one of the nodes relative to the second item;
- determining the spatial configuration of the network according to the method for determining a spatial configuration of a network as herein described;
- determining the relative spatial configuration in dependence of the determined spatial configuration of the network.

Like in the case of the method for determining a spatial configuration of a wireless inductive network as described above, also here, one or more further active nodes and/or one or more passive nodes in addition to the first and second passive nodes can be comprised in the network.

The positioning of a node relative to an item can in particular comprise mounting the node on the item. And/or it can comprise fixing the node relative to the item, or more specifically, fixing the node to the item.

The nodes can in particular be positioned at different locations, e.g., they can be mounted on the respective item at different locations.

The method can in addition comprise positioning at least still another one of the nodes relative to the first item or relative to the second item. This way the active node and the two passive nodes are positioned relative to one of the items.

In some embodiments, one or more of the nodes are mounted on, e.g., fixed to, a positioning device, and for at least one of the nodes, positioning the respective node relative to the respective item comprises mounting the positioning device on the respective item. The fixing device can in particular be deformable.

A flexibly deformable positioning device can facilitate positioning the nodes. For example, it can be wound around the respective item or around a part thereof.

The positioning device can in particular comprise one or more of: a fabric, a foil, a garment, a piece of clothing, a plaster, a sticky tape.

In some embodiments, the positioning device can be sticky. E.g., the positioning device can comprise a sticky member, such as a plaster or a sticky tape.

If two or more nodes, e.g., all nodes of the network are mounted on the positioning device, positioning the nodes relative to the respective item can comprise mounting the positioning device on one or both of: the first item and the second item.

For example, all passive nodes can be mounted on the positioning device, and positioning the passive nodes relative to the respective items can comprise mounting the positioning device on the first item and on the second item.

In some embodiments, the first item is one of:
- a human body or a part thereof;
- an animal body or a part thereof;
- a robot body or a part thereof;
- a carrier for use inside a human body;
- a carrier for use inside an animal body; and
the second item is one of:
- a human body or a part thereof;
- an animal body or a part thereof;
- a robot body or a part thereof;
- a carrier for use inside a human body;
- a carrier for use inside an animal body.

For example, the first and second items, are different parts of one and the same human body.

In another example, the first and second items, are different parts of one and the same animal body.

In another example, the first and second items, are different parts of one and the same robot body.

In another example, the first item is a human body, and the second item is a robot body.

In another example, the first item is a human body, and the second item is a carrier for use inside a human body.

In another example, the first item is an animal body, and the second item is a carrier for use inside an animal body.

For the carrier for use inside a human body and for the carrier for use inside an animal body can apply:
In some embodiments, it is a carrier for intracorporal use (in human and in animal bodies, respectively).

In some embodiments, it comprises bio-compatible material, in particular on its outside.

In some embodiments, it comprises a capsule.

In some embodiments, it is injectable. In some embodiments, it is implantable. In some embodiments, it is swallowable.

In some embodiments, it encloses one or more passive nodes and encloses no active node. In some embodiments, it encloses one node, and that node is a passive node, and encloses no active node.

Turning to specific apparatus aspects:
The combination for determining a spatial configuration of a wireless inductive network comprises
- the network, the network comprising at least three nodes, the nodes comprising an active node and at least two passive nodes comprising a first passive node and a second passive node; and
- a measuring unit operable for generating a probe signal in the active node to produce a probe electromagnetic field to contribute, by inductive interaction in response to the probe electromagnetic field involving the active node, the first passive node and the second passive node, to a back-coupled electromagnetic field to superpose in the active node with the probe signal to result in a measurement signal.

Furthermore,
- the first passive node can comprise a first oscillating circuit exhibiting a resonance behavior characterized by a first resonance curve comprising a first resonance peak at a first resonance frequency; and
- the second passive node can comprise a second oscillating circuit exhibiting a resonance behavior characterized by a second resonance curve comprising a second resonance peak at a second resonance frequency.

And more particularly, the first and second resonance peaks can overlap one another. This can increase the likelihood that a probe signal can effect inductive interaction involving the active node, the first passive node and the second passive node.

Furthermore, in some embodiments, the probe signal comprises an excitation frequency component, the excitation frequency component having an excitation frequency lying within one or both of: the first resonance peak and the second resonance peak. This can increase the likelihood that a probe signal can effect inductive interaction involving the active node, the first passive node and the second passive node.

The combination can be considered a system.

The combination can also be, e.g., a combination for determining a relative spatial configuration of a first item and a second item.

The active node can be connectable to the measurement unit; more particularly, it can be connected to the measurement unit. E.g., the active node can comprise a connector for that purpose. Also the measurement unit can comprise a connector for that purpose, to cooperate with the connector of the active node.

In some embodiments, the combination comprises a positioning device, in particular a deformable positioning device, and one or more of the nodes are mounted on, e.g., fixed to, the positioning device.

In some embodiments, the measurement unit is, in addition, operable
- for measuring one or more properties of the measurement signal to determine a measurement value for each of the properties; and
- for deriving measurement data from the so-determined one or more measurement values.

In some embodiments, the measurement unit furthermore comprises a data storage unit in which calibration data are stored, the calibration data comprising, for each of a plurality of calibration spatial configurations of the network, calibration measurement data associated with a respective calibration spatial configuration.

In some embodiments, the measurement unit furthermore is, in addition, operable for carrying out a classification step in which the spatial configuration is classified with the aid of the calibration data and in dependence of the measurement data. In particular, the classification step can comprise
- selecting, in dependence of the measurement data, one of the calibration measurement data;
- classifying the spatial configuration in dependence of a calibration spatial configuration which is associated with the selected calibration measurement data;
more particularly, the classifying step can comprise
- classifying the spatial configuration to be the calibration spatial configuration which is associated with the selected calibration measurement data.

In some embodiments, the measurement unit is operable for determining the spatial configuration in dependence of the measurement data, and the determining the spatial configuration in dependence of the measurement data comprises carrying out the classification step.

The measurement unit can be embodied as a single physical entity or can be distributed over two or more physical entities. E.g., it can comprise
- an impedance analyzer for generating a probe signal and for measuring the one or more properties of the measurement signal and for determining the measurement value for each of the properties; and
- a separate computing unit for deriving the measurement data from the measurement values and for earring out the classification step.

The separate computing unit can be, e.g., a computing device such as a desktop or laptop computer or a mobile computing device such as a smartphone or a tablet computer.

As will be readily understood, features mentioned herein with respect to a method can analogously apply for a described apparatus (combination) as well. And, vice versa, features mentioned herein with respect to an apparatus (combination) can analogously apply for a described method as well. The achievable effects correspond to one another.

As will have become clear from the above, the described invention can make possible to reduce the solution of a high-dimensional problem (determination of the spatial configuration of three or more nodes) to a lower-dimensional problem, namely to the measurement data, while nevertheless achieving good results, in particular when using classification.

Further embodiments and advantages emerge from the following description and the enclosed figures and from the dependent claims.

Below, the invention is described in more detail by means of examples and the included drawings. In the drawings, same reference numerals refer to same or analogous elements. The figures show schematically:
- Fig. 1: a diagrammatical illustration of a combination comprising a measuring unit and a network comprising an active node and two passive nodes;
- Fig. 2: a schematic illustration of different poses of a person with applied inductive network;
- Fig. 3: a strongly schematized illustration of a spatial configuration of a network and of inductive interactions;
- Fig. 4: a strongly schematized illustration of a spatial configuration of a network and of inductive interactions;
- Fig. 5: a strongly schematized illustration of a spatial configuration of a network and of inductive interactions;
- Fig. 6: a schematic illustration of two resonance curves of passive nodes of a network.

The described embodiments are meant as examples or for clarifying the invention and shall not limit the invention.

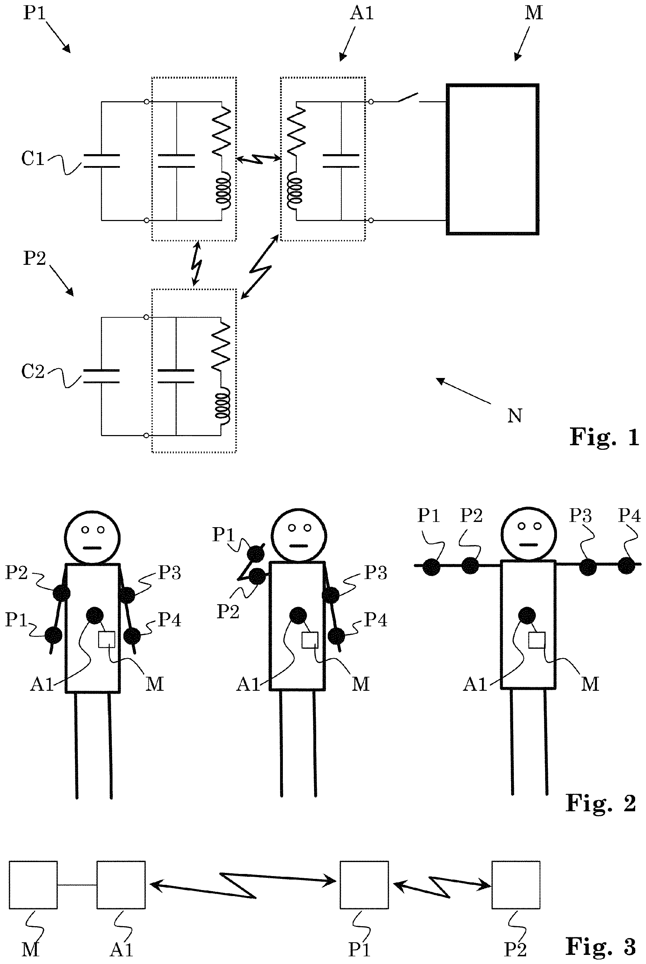

Fig. 1 shows a diagrammatical illustration of a combination comprising a measuring unit M and a network N comprising an active node A1 and two passive nodes PI, P2. Each of the nodes is an oscillating circuit comprising a coil symbolized in Fig. 1 by the contents of a dotted rectangle. Each of the coils has an inductance, a capacitance and an ohmic resistance, as illustrated in Fig. 1 in the dotted rectangles. Passive nodes PI, P2 each comprise a capacitor C1 and C2, respectively, for suitably selecting a resonance frequency of the respective oscillating circuit. Also the active node can comprise a capacitor for this purpose; not illustrated in Fig. 1.

The measuring unit M can comprise a signal generator, such as an oscillator, to produce a probe signal which is applied to active node A1. The probe signal can be, e.g., a sine voltage of constant amplitude having a frequency (excitation frequency) of, e.g., 500 kHz. The probe signal can have a duration of, e.g., between 50 ms and 1 s.

In response to application of the probe signal, a probe electromagnetic field is produced by active node A1.

As illustrated by the double-ended arrows, the probe electromagnetic field inductively (and wirelessly) interacts with passive nodes P1 and P2, and also passive nodes P1 and P2 can inductively (and wirelessly) interact in response to the probe electromagnetic field. The inductive interaction involves active node A1, first passive node P1 and second passive node P2 and contribute to a back-coupled electromagnetic field. In case no further nodes interact in this inductive interaction, as in the illustrated case of Fig. 1, the inductive interaction not only contributes to the back-coupled electromagnetic field, but produces the back-coupled electromagnetic field.

The back-coupled electromagnetic field superposes with the probe electromagnetic field and induces in active node A1 a back-coupled signal. The back-coupled signal superposes in active node A1 with the probe signal to result in a measurement signal. The measurement signal can be the current signal at active node A1, more specifically, at its coil.

By measurement unit M, e.g., an amplitude of the measurement signal (current amplitude) and/or a phase shift of the measurement signal (current signal) relative to the voltage signal (probe signal) can be measured as properties of the measurement signal. From the measured values (measurement values), such as a figure of amperes, e.g., 1.2 mA, and a figure of degrees, e.g., 24.7°, measurement data can be derived, e.g., in a trivial way, such as by simply taking the measurement values; the measurement data in that case being, e.g., (1.2 mA; 24.7°).

The measurement data can be used to determine the spatial configuration of the network, e.g., the relative positions of the nodes.

Fig. 2 shows a schematical illustration of three different poses of a person with applied inductive network - to further illustrate and explain the invention. The network illustrated in Fig. 2 comprises an active node A1 and four passive nodes P1 to P4, all symbolized by filled circles.

Active node A1 is mounted on the person's chest, passive node P1 on the person's right wrist, passive node P2 on the person's right upper arm, passive node P3 on the person's left upper arm, and passive node P4 on the person's left wrist. For example, passive nodes P1 to P4 and optionally also active node A1 can be mounted on a jacket the person is wearing. Or the nodes are mounted on a band each encompassing the respective part of the person's body (wrist, upper arm, chest).

In the pose illustrated in the left third of Fig. 2, both arms are hanging, in the pose illustrated in the middle third of Fig. 2, the left arm is hanging, whereas the right arm is lifted and bent, such as for telephoning, and in the pose illustrated in the right third of Fig. 2, both arms are held horizontally.

If we assume that in the network of Fig. 2, like in case of the network of Fig. 1, only active node A1 and the two passive nodes PI, P2 are inductively interacting, e.g., because the other passive nodes (P3, P4) have resonance frequencies much different from the excitation frequency, it is clear that the inductive interaction is likely to be very different for the three different poses, as at least some of the distances between the nodes are different for the poses. (Note: The distance between passive node P1 and passive node P2 is identical for the left pose and the right pose, but for each of the passive nodes PI, P2, the distance to active node A1 is different for the left pose and the right pose.)

Thus, it can be expected that the measurement signals and the measurement data for all three poses differ from one another. Accordingly, it can be possible to determine the pose from the measurement data. For example, calibration data can be taken, determining the measurement signals and the measurement data for all three poses and assigning each of the three poses to the respective measurement data. When the person moves into an unknown pose, that pose can be classified to be one of the three calibration poses, based on the measurement data determined in the unknown pose.

For more detailed information about an unknown pose, the further two passive nodes P3, P4 can be used, e.g., by subsequent application to active node A1 of a further probe signal of a suitable frequency (excitation frequency), different from the excitation frequency used for passive nodes PI, P2 which would not contribute to the back-coupled signal at that frequency. Alternatively, the further probe signal could be applied to a second active node (not illustrated in Fig. 2).

This way, two measurements are required (at different excitation frequencies) for more detailed information about an unknown pose, the measurements taking place one after the other (if both probe signals are applied to active node A1) or optionally taking place simultaneously, in case the further probe signal is applied to the second active node - using the same measurement unit or a different one.

However, it is also possible to derive more detailed information about an unknown pose from only a single measurement, namely if three of the passive nodes or all four passive nodes P1 to P4 interact inductively in response to the probe signal. Of course such an interaction between four or five nodes and oscillating circuits, respectively, are very complex, but the evaluation of the corresponding measurement data can be quick and simple, e.g., based on calibration data and classification.

Figs. 3 to 5 each show a strongly schematized illustration of a spatial configuration of a network and of inductive interactions. This illustrates, in a strongly schematized way, inductive interactions and related effects which arise already in case of having interaction between three nodes (one active node A1 and two passive nodes PI, P2) instead of only two.

In Fig. 3 is illustrated the case that passive node P2 is located too far away from active node A1 and/or unsuitably oriented relative to active node A1 for direct inductive interaction with active node A1, more specifically, for directly contributing to the measurement data. However, the provision of passive node P1 can effect that the presence of passive node P2 does influence the inductive interaction and, consequently, also influences the measurement data - because of inductive interaction passive node P1 and passive node P2.

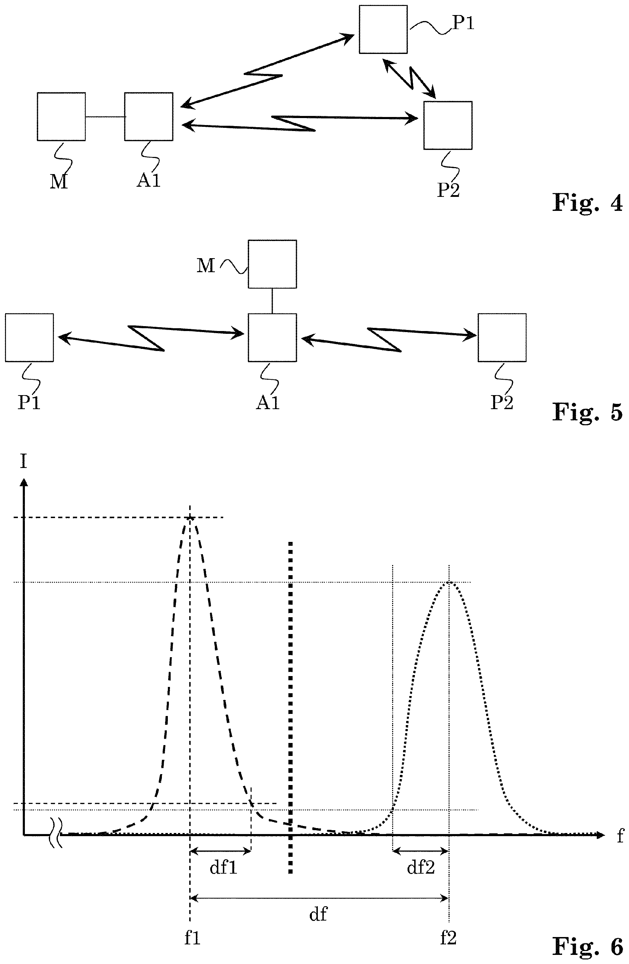

In Fig. 4 is illustrated the case that the two passive nodes PI, P2 strongly interact inductively, which again effects active node A1, thus influencing the measurement data - in a different way than in case both passive nodes PI, P2 would merely separately inductively interact with active node A1.

In Fig. 5 is illustrated the relatively simple case that the two passive nodes PI, P2 inductively interact with active node, but in a relatively independent fashion, i.e. with no (or negligible) direct inductive interaction between passive nodes PI, P2.

As has been mentioned before, the resonance frequencies of the passive nodes can influence whether or not they take part in the inductive interaction. It can be advantageous if their resonance peaks overlap.

Fig. 6 shows a schematic illustration of two resonance curves of passive nodes of a network with one active and two passive nodes. A possible definition of overlapping resonance peaks shall be explained by means of Fig. 6. An alternative definition based on certain dB widths can analogously be applied, but do not take into account effect which can arise in case of (strongly) asymmetric peak shapes.

Resonance curves are well known and well defined. Of course, a resonance curve is determined for a separate node, without inductive interaction with further nodes.

For example, a sine-shaped voltage of constant amplitude and variable frequency is applied to the oscillating circuit, and the resulting frequency-dependent current amplitude is monitored. Accordingly, the x-axis is the frequency axis, and the y-axis is the current amplitude axis.

The dashed curve is the resonance curve of a first passive node, the dotted curve is the resonance curve of a second passive node. The first passive node has a resonance frequency f1 which is smaller than the resonance frequency f2 of the second passive node. The upper dashed and dotted straight lines, respectively, indicate the maximum current which flows at the respective resonance frequency for the first passive node and the second passive node, respectively.

Fig. 6 illustrates the case of a 10 %-half-width, corresponding to a 20 dB width being used - in the definition of peak overlap. Accordingly, the lower dashed and dotted straight lines, respectively, indicate the level of 10 % of the maximum current for the first passive node and the second passive node, respectively. The upper half width of the resonance peak at f1 is indicated as df1, the lower half width of the resonance peak at f2 is indicated as df2. If the difference df of the two resonance frequencies is smaller than df1+df2, the peaks would be considered to overlap. Obviously, the resonance peaks in Fig. 6 do not overlap in this sense.

Furthermore, the thick dotted vertical line in Fig. 6 indicates an excitation frequency. It can, in instances, increase the likelyhood that inductive interaction between three nodes (active node and first and second passive nodes) takes place and influences the measurement data if the excitation frequency lies within one or both of the resonance peaks.

Also a definition of a specific frequency (here: the excitation frequency) lying within a resonance peak shall be explained by means of Fig. 6: E.g., a frequency lies within a resonance peak if the resonance curve assumes at the specific frequency a value of at least a certain percentage of a value of the resonance curve at the frequency. The percentage can be, e.g., 1 %, more particularly 3.162 %, still more particularly 10 %.

In the example of Fig. 6, the current of the resonance curve of the first passive node at the excitation frequency amounts to between 1 % and 10 % of the maximum current of the resonance curve. Thus, if the percentage is selected to be 1 %, the excitation frequency is considered to lie within the resonance peak af f1. But the current of the resonance curve of the second passive node at the excitation frequency is very low, e.g., below 1/10000 (0.01 %), so that the excitation frequency will be considered not to lie within the second resonance peak at f2.

## Claims

1. Method for determining a spatial configuration of a network which is a wireless inductive network comprising at least three nodes, the nodes comprising an active node and at least two passive nodes comprising a first passive node and a second passive node, the method comprising
- generating a probe signal in the active node producing a probe electromagnetic field;
- inductive interaction in response to the probe electromagnetic field contributing to a back-coupled electromagnetic field, the inductive interaction involving the active node, the first passive node and the second passive node;
- the back-coupled electromagnetic field superposing with the probe electromagnetic field and inducing in the active node a back-coupled signal;
- the back-coupled signal superposing in the active node with the probe signal to result in a measurement signal;
- measuring one or more properties of the measurement signal to determine a measurement value for each of the properties;
- deriving measurement data from the so-determined one or more measurement values;
- determining the spatial configuration in dependence of the measurement data; in particular wherein each of the nodes comprises an oscillating circuit.

2. The method according to claim 1, wherein the measurement signal depends on a presence of both, the first and the second passive node, more particularly, wherein the measurement data depend on a presence of both, the first passive node and the second passive node.

3. The method according to claim 1 or claim 2, wherein inductive interaction between the first passive node and the second passive node in response to the probe electromagnetic field contributes to the back-coupled electromagnetic field.

4. The method according to one of claims 1 to 3, wherein
- inductive interaction between the active node and the first passive node in response to the probe electromagnetic field; and
- inductive interaction between the active node and the second passive node in response to the probe electromagnetic field
contribute to a back-coupled electromagnetic field.

5. The method according to one of claims 1 to 4, wherein
- the first passive node comprises a first oscillating circuit exhibiting a resonance behavior **characterized by** a first resonance curve comprising a first resonance peak at a first resonance frequency; and
- the second passive node comprises a second oscillating circuit exhibiting a resonance behavior **characterized by** a second resonance curve comprising a second resonance peak at a second resonance frequency;
wherein the first and second resonance peaks overlap one another.

6. The method according to claim 5, the probe signal comprising an excitation frequency component, the excitation frequency component having an excitation frequency lying within one or both of: the first resonance peak and the second resonance peak.

7. The method according to claim 5 or claim 6, wherein twice a difference between the first resonance frequency and the second resonance frequency is identical to or smaller than a sum of a 40 dB width of the first resonance peak and a 40 dB width of the second resonance peak, more particularly smaller than a sum of a 30 dB width of the first resonance peak and a 30 dB width of the second resonance peak, still more particularly smaller than a sum of a 20 dB width of the first resonance peak and a 20 dB width of the second resonance peak.

8. The method according to one of claims 5 to claim 7, wherein the first resonance frequency is lower than the second resonance frequency, and wherein a difference between the first resonance frequency and the second resonance frequency is identical to or smaller than a sum of an upper half-width of the first resonance peak and a lower half-width of the second resonance peak,
- the upper half-width of the first resonance peak being a difference between the first resonance frequency and a frequency larger than the first resonance frequency at which the first resonance curve assumes a value of 1 % of a value of the first resonance curve at the first resonance frequency;
- the lower half-width of the second resonance peak being a difference between the second resonance frequency and a frequency smaller than the second resonance frequency at which the second resonance curve assumes a value of 1 % of a value of the second resonance curve at the second resonance frequency;
more particularly, wherein
- the upper half-width of the first resonance peak being a difference between the first resonance frequency and a frequency larger than the first resonance frequency at which the first resonance curve assumes a value of 10 % of a value of the first resonance curve at the first resonance frequency;
- the lower half-width of the second resonance peak being a difference between the second resonance frequency and a frequency smaller than the second resonance frequency at which the second resonance curve assumes a value of 10 % of a value of the second resonance curve at the second resonance frequency.

9. The method according to one of claims 1 to claim 8, the method further comprising
- providing calibration data comprising, for each of a plurality of calibration spatial configurations of the network, calibration measurement data associated with a respective calibration spatial configuration;
wherein determining the spatial configuration in dependence of the measurement data comprises
- carrying out a classification step in which the spatial configuration is classified with the aid of the calibration data and in dependence of the measurement data.

10. Method for determining a relative spatial configuration of a first item and a second item, the method comprising
- providing a network which is a wireless inductive network comprising at least three nodes, the nodes comprising an active node and at least two passive nodes comprising a first passive node and a second passive node;
- positioning at least one of the nodes relative to the first item;
- positioning at least another one of the nodes relative to the second item;
- determining the spatial configuration of the network according to the method of one of claims 1 to 9;
- determining the relative spatial configuration in dependence of the determined spatial configuration of the network.

11. The method according to claim 10, wherein one or more of the nodes are mounted on a deformable positioning device, and for at least one of the nodes, positioning the respective node relative to the respective item comprises mounting the positioning device on the respective item; in particular wherein the positioning device comprises one or more of: a fabric, a foil, a garment, a piece of clothing, a plaster, a sticky tape.

12. The method according to claim 10 or claim 11, wherein the first item is one of:
- a human body or a part thereof;
- an animal body or a part thereof;
- a robot body or a part thereof;
- a carrier for use inside a human body;
- a carrier for use inside an animal body; and
the second item is one of:
- a human body or a part thereof;
- an animal body or a part thereof;
- a robot body or a part thereof;
- a carrier for use inside a human body;
- a carrier for use inside an animal body.

13. Combination for determining a spatial configuration of a network which is a wireless inductive network, the combination comprising
- the network, the network comprising at least three nodes, the nodes comprising an active node and at least two passive nodes comprising a first passive node and a second passive node; and
- a measuring unit operable for generating a probe signal in the active node to produce a probe electromagnetic field to contribute, by inductive interaction in response to the probe electromagnetic field involving the active node, the first passive node and the second passive node, to a back-coupled electromagnetic field to superpose in the active node with the probe signal to result in a measurement signal; and
wherein
- the first passive node comprises a first oscillating circuit exhibiting a resonance behavior **characterized by** a first resonance curve comprising a first resonance peak at a first resonance frequency; and
- the second passive node comprises a second oscillating circuit exhibiting a resonance behavior **characterized by** a second resonance curve comprising a second resonance peak at a second resonance frequency;
wherein the first and second resonance peaks overlap one another; and
in particular wherein the probe signal comprises an excitation frequency component, the excitation frequency component having an excitation frequency lying within one or both of: the first resonance peak and the second resonance peak.

14. The combination according to claim 13, wherein the measurement unit is, in addition, operable
- for measuring one or more properties of the measurement signal to determine a measurement value for each of the properties; and
- for deriving measurement data from the so-determined one or more measurement values;
the measurement unit comprising a data storage unit in which calibration data are stored, the calibration data comprising, for each of a plurality of calibration spatial configurations of the network, calibration measurement data associated with a respective calibration spatial configuration.

15. The combination according to claim 14, wherein the measurement unit is, in addition, operable for carrying out a classification step in which the spatial configuration is classified with the aid of the calibration data and in dependence of the measurement data, in particular wherein the calibration step comprises
- selecting, in dependence of the measurement data, one of the calibration measurement data;
- classifying the spatial configuration in dependence of a calibration spatial configuration which is associated with the selected calibration measurement data, more particularly classifying the spatial configuration to be the calibration spatial configuration which is associated with the selected calibration measurement data.
